(19) Europäisches Patentamt
European Patent Office
Office européen des brevets

(11) **EP 0 899 331 B1**

(12) **FASCICULE DE BREVET EUROPEEN**

(45) Date de publication et mention
de la délivrance du brevet:
**21.02.2007   Bulletin 2007/08**

(51) Int Cl.:
*C12N 9/76* *(2006.01)*      *A23J 3/34* *(2006.01)*

(21) Numéro de dépôt: **97202591.0**

(22) Date de dépôt: **22.08.1997**

(54) **Enzyme protéolytique purifiée et procédé de purification**

Gereinigtes proteolytisches Enzym und Verfahren zur Reinigung

Purified proteolytic enzyme and procedure for purification

(84) Etats contractants désignés:
**AT BE CH DE DK ES FI FR GB GR IE IT LI LU NL PT SE**

(43) Date de publication de la demande:
**03.03.1999   Bulletin 1999/09**

(73) Titulaire: **SOCIETE DES PRODUITS NESTLE S.A.**
**1800 Vevey (CH)**

(72) Inventeurs:
• **Braun, Marcel**
**3510 Konolfingen (CH)**
• **Neumann, Fred**
**3612 Steffisburg (CH)**

(74) Mandataire: **Elleby, Gudrun et al**
**Nestec S.A.**
**IP Department**
**Avenue Nestlé, 55**
**1800 Vevey (CH)**

(56) Documents cités:
**WO-A-90/13638          WO-A1-86/03774**
**US-A- 3 886 043          US-A- 4 136 201**

• **AUSUBEL F.M.: "Current Protocols in Molecular Biology" 1995 , WILEY & SONS, NEW YORK, USA XP002054528 * page .A.3F.12 ***

**Description**

L'invention concerne une enzyme protéolytique purifiée et un procédé de purification d'une enzyme protéolytique, notamment de trypsine.

Les protéases commerciales, en particulier la trypsine commerciale, même après purification par traitement spécial, par exemple par double cristallisation, contient des lipases résiduelles, en particulier la phospholipase A2, particulièrement résistante à la désactivation thermique que l'on fait subir à la protéase après son utilisation dans un processus d'hydrolyse.

Dans la fabrication des hydrolysats de protéine, en particulier destinés à entrer dans la composition de produits infantiles, on utilise couramment la trypsine. Pour incorporer la partie protéique dans un produit fini, par exemple un lait infantile, il faut supprimer toute activité enzymatique lipolytique résiduelle provenant de l'hydrolysat protéique. Celà est nécessaire pour éviter l'apparition de produits de dégradation de la lécithine que l'on ajoute à la formule finale pour des raisons technologiques, par exemple pour améliorer la mouillabilité des poudres, en lysolécithine, en particulier durant l'entreposage. De tels produits de dégradation peuvent se manisfester aussi bien dans les produits liquides que dans les poudres par l'apparition de défauts de stabilité ou organoleptiques, par exemple de taches, de mauvais goûts, ou par leur toxicité conduisant à des effets secondaires, par exemple de type inflammatoire chez les nourrissons.

Or, il se trouve que l'élimination complète des phospholipases en particulier est difficile à réaliser. La purification complète des protéases requiert généralement différentes étapes de précipitation, des séparations chromatographiques, des traitements thermiques dans des conditions bien définies ou des inactivations par voie chimique. L'élimination complète de la phospholipase A2, qui est très thermorésistante, nécessite un traitement thermique prolongé qui affecte malheureusement également la protéase.

Le but de l'invention est la préparation d'une protéase purifiée dont l'activité protéolytique soit préservée quantitativement et qualitativement, mais qui soit exempte d'activité lipolytique, notamment de phospholipase A2, par un procédé simple et économique.

On connait un procédé de préparation de trypsine purifiée, décrit par exemple dans US-A-3886043, dans lequel on réalise la chromatographie d'une solution tampon de trypsine cristallisée par passage sur une résine constituée de gel de dextrane avec groupes sulfoniques greffés, dans le but de séparer les différentes formes actives de trypsine porcine.

On sait également préparer une présure microbienne exempte de lipase, par exemple par le procédé décrit dans US-A-4136201, par culture de Mucor miehei sur un milieu nutritif approprié.

L'invention concerne une préparation enzymatique protéolytique purifiée, caractérisée par le fait qu'elle possède une activité phospholipase A2 résiduelle d'au plus 20 mU/g d'enzyme pure détectable par analyse par chromatographie à haute performance des phospholipides après incubation avec une formule infantile dont l'activité phospholipase A2 n'est pas détectable et que son activité protéase est maintenue à au moins 75 % de l'activité initiale de l'enzyme.

Les mesures des activités enzymatiques sont détaillées dans les exemples ci-après. En particulier, on entend par "non détectable" une activité phospholipase A2 résiduelle < 6 mU/g d'enzyme.

L'enzyme peut être toute protéase d'origine végétale, microbienne ou animale ou d'origine biogénétique. C'est de préférence une protéase d'origine animale, telle que la pancréatine, particulièrement la trypsine d'origine porcine.

Le procédé selon l'invention est caractérisé par le fait:

1) Que l'on ajuste de pH d'une solution de la protéase à une valeur comprise entre 6 et 9 et que l'on maintient la solution à ce pH et à 20-35° C pendant au moins 15 min et au plus 120 min, de manière à utiliser l'activité protéolytique de la protéase pour détruire l'activité lipolytique des lipases et des phospholipases du milieu réactionnel et
2) Que l'on abaisse le pH de la solution à une valeur inférieure ou égale à 3,5, l'ordre des étapes 1) et 2) précédentes pouvant être inversé.

Dans un mode de réalisation préféré, permettant de supprimer également les traces de lipases résiduelles autres que la phospholipase A2, le procédé comporte une étape finale de traitement thermique, de préférence par UHT. Les traces de lipases thermosensibles sont ainsi éliminées.

De préférence, l'ajustement du pH vers le domaine alcalin a lieu avant l'abaissement du pH dans le domaine acide, dans la mesure où l'on peut ainsi différer l'utilisation de la protéase. Dans la variante où les deux étapes sont inversées, il faut utiliser la protéase immédiatement après le traitement.

Dans une mise en oeuvre préférée, on ajoute au milieu réactionnel un sel de magnésium soluble dans celui-ci, de préférence en début de réaction, ce qui permet de stabiliser les protéases tout en favorisant la dégradation des phospholipases. On ajoute de préférence du chlorure de magnésium à raison de 10 à 200 mM/l de milieu réactionnel, par exemple 50 à 100 mM/l de milieu réactionnel.

La concentration de protéase pure dans la solution avant traitement peut être comprise entre 0,5 et 6 %, et est de préférence environ 2,5 % en poids.

L'invention concerne également un procédé de préparation d'un aliment infantile à base d'hydrolysat de protéine, caractérisé par le fait que l'on hydrolyse enzymatiquement un produit lactosérique au moyen d'une protéase purifiée précédente, que l'on traite l'hydrolysat à 75-85° C/3-5 min., qu'on y ajoute de la matière grasse liquide et des minéraux, qu'on effectue un traitement UHT à 125-135° C/2-3 min., puis que l'on y ajoute des hydrates de carbone, des vitamines et des oligoéléments, que l'on stérilise par UHT et que l'on conditionne aseptiquement le produit liquide.

Selon une variante de ce procédé, on sèche le liquide, notamment par pulvérisation après le traitement de stérilisation par UHT.

L'enzyme purifiée selon l'invention peut être utilisée en dehors du domaine alimentaire dans les applications des protéases, par exemple dans la préparation d'une composition nutritionelle, cosmétique ou pharmaceutique.

On peut citer à cet égard les applications anti-inflammatoires, le traitement des troubles de la digestion, le traitement des thromboses, le soin des blessures et plaies et l'élimination des tissus nécrosés par exemple.

Les exemples ci-après illustrent l'invention. Dans ceux-ci, les parties et pourcentages sont en poids, sauf indication contraire.

**Exemple 1**

On met en solution 1 kg de trypsine porcine 6.0 S du commerce (Novo, Danemark) dans 10 kg d'eau déminéralisée à 25° C sous agitation dans un récipient, la concentration en protéase étant 9,1 % et le pH initial 5. Afin d'être assuré de ne pas transporter de protéase non dissoute dans l'étape suivante, on transfère la solution dans un nouveau récipient.

On ajoute une solution aqueuse diluée de NaOH à 1 M/l pour ajuster le pH de la solution à 8. On garde ensuite le pH constant pendant 15 min. par addition, selon besoin, de la solution aqueuse de NaOH précédente, par exemple au moyen d'un pH-stat, sous agitation, afin d'hydrolyser les phospholipases.

Après ce traitement, on stabilise les protéases, c'est à dire la trypsine et la chymotrypsine en abaissant le pH du milieu réactionnel à 3 par addition d'une solution aqueuse de HCl à 1M/l. On peut utiliser la solution de protéases immédiatement dans une réaction d'hydrolyse ou l'entreposer, par exemple à -25° C en vue d'une utilisation différée.

Les analyses ci-après montrent l'activité enzymatique de l'enzyme protéolytique purifiée obtenue selon l'invention en comparaison avec celle de l'enzyme cristallisée du commerce (trypsine PTN 6.0 S, Novo, Danemark) de départ n'ayant pas subi le traitement selon l'invention:

1. Détermination des phospholipases:

1.1 Détermination de la phospholipase A2 par méthode radioisotopique.
La méthode est basée sur le clivage de phosphatidyl-choline (C14-dioleyl) par la phospholipase A2 et la détection radiométrique ponctuelle des fractions marquées après séparation chromatographique.
1.2 Détermination des phospholipases totales par titrimétrie.
La méthode n'est pas spécifique de la phospholipase A2 et détecte toutes les phospholipases. Elle est basée sur la titration des acides gras libérés à partir des phospholipides de jaune d'oeuf (Fluka, Buchs, Suisse) par les phospholipases à pH 8 (maintenu par pH-stat) et à température constante de 40° C avec 1,4 mM de sodiumdéoxycholate et 3 mM $CaCl_2$. On utilise 2 g de phospholipide de jaune d'oeuf purifié avec addition de 250 mg d'inhibiteur de trypsine de blanc d'oeuf de dinde (Sigma, St. Louis, USA) pour 1 g de trypsine.

2. Détermination de lipase et estérase:

2.1 On détermine l'activité des lipases par titrimétrie en utilisant l'huile d'olive comme substrat à raison de 100 g/l à pH constant de 8,9 (pH-stat) en présence de 1,25 g/l de taurocholate et de 82,5 g/l de gomme arabique.

2.2 On détermine l'activité des estérases en utilisant la méthode précédente (2.1) mais en prenant les triglycérides à chaîne moyenne (MCT) comme substrat.

3. Détermination des protéases:

3.1. Pour la trypsine, on utilise la méthode décrite par Erlanger et coll. dans Arch. Biochem. Biophys. 95, 271-278.

3.2. Pour la chymotrypsine, on utilise la méthode de l'US Pharmacopeia XXI (1985).

Les résultats des analyses d'activités dans la préparation d'enzyme sont indiqués dans le tableau 1 ci-après:

**Tableau 1**

| Prépara-Tion d'enzyme | Phospholipase A2 (U/g), 1.1 | Phospholipases (U/g) | Lipases (U/g) | Estérase (U/g) | Trypsine (g/kg) | Chymotrypsine (USP/mg) |
|---|---|---|---|---|---|---|
| Trypsine PTN 6.0 S purifiée selon l'invention | <0,0022 | 0,79 | O,21 | 0,22 | 223 | 41 |
| Trypsine PTN 6.0 S de départ | 87 | 14 | 0,34 | 0,39 | 213 | 42 |

On constate que le traitement permet de diminuer considérablement l'activité des enzymes autres que les protéases, en particulier de supprimer celle de la phospholipase A2, tout en maintenant intacte l'activité protéolytique en qualité et en quantité, en particulier l'équilibre entre la trypsine (93 % d'activité relativement à l'enzyme non traitée) et la chymotrypsine (86 % d'activité relativement à l'enzyme non traitée).

**Exemple 2**

On met en solution 1 kg de trypsine porcine 6.0 S du commerce (Novo, Danemark) dans 10 kg d'eau déminéralisée à 25° C sous agitation dans un récipient, la concentration en protéase étant 9,1 % et le pH initial 5. Afin d'être assuré de ne pas transporter de protéase non dissoute dans l'étape suivante, on transfère la solution dans un nouveau récipient.

On ajoute 224 g de chlorure de magnésium (MgCl2 * 6 H2O), on ajuste de pH à 8,5 en 15 min. avec une solution aqueuse diluée de NaOH à 1 M/l. On laisse réagir le milieu pendant 120 min. à 25° C sans contrôler le pH, afin d'hydrolyser les phospholipases.

Après ce traitement, on stabilise les protéases, c'est à dire la trypsine et la chymotrypsine en abaissant le pH du milieu réactionnel à 3 par addition d'une solution aqueuse de HCl à 1M/l et on laisse la solution au repos pendant 16 h à 4° C. La solution de trypsine purifiée est alors prête à être utilisée.

On constate que l'activité relative de la trypsine est 93 % de celle de départ et que l'activité relative de la chymotrypsione est 86 % de celle de départ.

**Exemple 3**

On utilise la préparation enzymatique purifiée de l'exemple 2 pour préparer une formule infantile hypoallergénique. On réalise l'hydrolyse de protéines de lactosérum, puis on traite l'hydrolysat à 75-85° C/3-5 min., on y ajoute de la matière grasse et des minéraux, on effectue un traitement UHT à 125° C/2 min., puis on ajoute de la maltodextrine et des vitamines, on stérilise par UHT à 148° C/5 s et on conditionne aseptiquement le produit liquide.

Pour tester l'activité enzymatique résiduelle, on ajoute 10 ml de solution de trypsine purifiée selon l'exemple 1 à 100 ml de la formule infantile liquide précédente dont l'activité de la phospholipase A2 est < 6mU/g trypsine. Après mélange, on réduit les activités des lipase et estérase par un traitement thermique à 75-85° C/3-5 min. en bain d'eau. Après refroidissement à la température ambiante, on ajoute 55 mg d'azidure de sodium et on incube la solution à 40° C/4 j. Après incubation, on analyse la composition des phospholipides par chromatographie liquide haute performance (HPLC) et on calcule l'activité de la phospholipase A2, exprimée en mU/100 g de produit (mU PL-A2), à partir des différences de concentration des lysophospholipides selon la formule:

$$mU\ PL\text{-}A2 =$$

$$\frac{(LPC+LPE)\ \text{au temps t2 moins }(LPC+LPE)\ \text{au temps t1 en mg/100 g de produit}}{540\ (\text{masse moléculaire des lysolécithines d'oeuf}) \times (t1 - t2)}$$

avec LPC = lysophosphatidyl choline et LPE = lysophosphatidyl éthanolamine, ainsi que cette valeur rapportée à la concentration de trypsine pure en g/100 g, soit mU PL-A2/g de trypsine pure.

On évalue également la dégradation de la trypsine et de la chymotrypsine en % par rapport à l'activité initiale, ainsi que la dégradation des phospholipides après 9 mois d'entreposage à 20° C en % des phospholipides de départ.

Les résultats sont indiqués dans le tableau 2 ci-après:

**Tableau 2**

| Formule infantile liquide | PL-A2 (mU/g de trypsine pure), HPLC | Trypsine (% de l'initiale) | Chymotrypsine (% de l'initiale) | Dégradation des phospholipides (% des initiaux) |
|---|---|---|---|---|
| Hydrolysée par la trypsine PTN 6.0 S purifiée selon l'invention | 16 | 94 | 75 | <1 |
| Hydrolysée par la trypsine PTN 6.0 S de départ | 349000 | 100 | 100 | 100 |

**Exemple 4**

On prépare un produit infantile comme à l'exemple 3, sauf que l'on ajoute un phospholipide au mélange liquide avant de le sécher par pulvérisation. On constate que l'activité des phospholipases est fortement liée à la dégradation des phospholipides du produit. De grands volumes de charges, une production interrompue, associées à une activité élevée des phospholipases dégrade les phospholipides jusqu'à un certain degré avant que le produit ne soit séché. Les produits contenant la trypsine purifiée selon l'invention montrent une dégradation nettement moindre des phospholipides ajoutés, selon la quantité totale des phospholipides ajoutés dans la formule, <1 %, alors qu'elle représente 20 à 90 % lorsque l'on utilise la même trypsine n'ayant pas subi le traitement de purification selon l'invention.

De plus, l'analyse des protéines résiduelles par SDS-PAGE et l'analyse des antigènes immunologiquement actifs par ELISA n'ont pas montré de différences significatives par utilisation de trypsine purifiée selon l'invention rapport à une production avec la trypsine non purifiée.

**Revendications**

1. Préparation enzymatique protéolytique purifiée, **caractérisée par le fait qu'**elle possède une activité phospholipase A2 résiduelle d'au plus 20 mU/g d'enzyme pure détectable par analyse par chromatographie à haute performance des phospholipides après incubation avec une formule infantile dont l'activité phospholipase A2 n'est pas détectable et que son activité protéase est maintenue à au moins 75 % de l'activité initiale de l'enzyme.

2. Préparation enzymatique selon la revendication 1, **caractérisée par le fait que** l'enzyme est la trypsine porcine.

3. Procédé de purification d'enzyme protéolytique, notamment de trypsine, **caractérisé par le fait:**

   1) **Que** l'on ajuste de pH d'une solution de la protéase à une valeur comprise entre 6 et 9 et que l'on maintient la solution à ce pH et à 20-35° C pendant au moins 15 min et au plus 120 min, de manière à utiliser l'activité protéolytique de la protéase pour détruire l'activité lipolytique des lipases et des phospholipases du milieu réactionnel et
   2) **Que** l'on abaisse le pH de la solution à une valeur inférieure ou égale à 3,5, l'ordre des étapes 1) et 2) précédentes pouvant être inversé.

**4.** Procédé selon le revendication 3, **caractérisé par le fait qu'**il comporte une étape finale de traitement thermique, de préférence par UHT, permettant de supprimer également les traces de lipases résiduelles autres que la phospholipase A2.

**5.** Procédé selon la revendication 3, **caractérisé par** le fait l'ajustement du pH entre 6 et 9 a lieu avant l'abaissement du pH à une valeur inférieure ou égale à 3,5.

**6.** Procédé selon la revendication 3, **caractérisé par le fait que** l'on ajoute au milieu réactionnel un sel de magnésium soluble dans celui-ci, de préférence en début de réaction, ce qui permet de stabiliser les protéases tout en favorisant la dégradation des phospholipases.

**7.** Procédé selon la revendication 6, **caractérisé par le fait que** l'on ajoute du chlorure de magnésium à raison de 10 à 200 mM/l et de préférence à raison de 50 à 100 mM/l de milieu réactionnel.

**8.** Procédé selon la revendication 3, **caractérisé par le fait que** la concentration de protéase pure dans la solution avant traitement est comprise entre 0,5 et 6 %, et est notamment environ 2,5 % en poids.

**9.** Procédé de préparation d'un aliment infantile à base d'hydrolysat de protéine, **caractérisé par le fait que** l'on hydrolyse enzymatiquement un produit lactosérique au moyen d'une protéase purifiée selon la revendication 1, que l'on traite l'hydrolysat à 75-85° C/3-5 min., qu'on y ajoute de la matière grasse liquide et des minéraux, qu'on effectue un traitement UHT à 125-135° C/2-3 min., puis que l'on y ajoute des hydrates de carbone, des vitamines et des oligoéléments, que l'on stérilise par UHT et que l'on conditionne aseptiquement le produit liquide.

**10.** Procédé selon la revendication 9, **caractérisé par le fait qu'**après le traitement de stérilisation par UHT, l'on sèche le liquide, notamment par pulvérisation.

**11.** Utilisation d'une préparation enzymatique purifiée selon la revendication 1, dans la préparation d'une composition nutritionnelle, cosmétique ou pharmaceutique.

**Claims**

**1.** A purified proteolytic enzymatic preparation **characterised in that** it possesses a residual phospholipase A2 activity of no more than 20 mU/g of pure enzyme detectable by high performance chromatography analysis of the phospholipids after incubation with an infant formula of which the phospholipase A2 activity is not detectable, and that its protease activity is maintained at at least 75% of the initial activity of the enzyme.

**2.** The enzymatic preparation according to claim 1, **characterised in that** the enzyme is porcine trypsin.

**3.** A process for the purification of proteolytic enzyme, particularly trypsin, **characterised in that**:

1) the pH of a solution of the protease is adjusted to a value of 6 to 9 and the solution is maintained at this pH and at 20-35°C for at least 15 min and a maximum of 120 min so as to utilise the proteolytic activity of the protease to destroy the lipolytic activity of the lipases and phospholipases of the reaction medium and
2) the pH of the solution is reduced to a value less than or equal to 3.5, it being possible to reverse the order of steps 1) and 2) above.

**4.** The process according to claim 3, **characterised in that** it includes a final heat treatment step, preferably by UHT, enabling the traces of residual lipases other than phospholipase A2 also to be removed.

**5.** The process according to claim 3, **characterised in** the adjustment of the pH to between 6 and 9 takes place before the reduction of the pH to a value less than or equal to 3.5.

**6.** The process according to claim 3, **characterised in that** a magnesium salt soluble in the reaction medium is added to the reaction medium, preferably at the beginning of the reaction, enabling the proteases to be stabilised while promoting the degradation of the phospholipases.

**7.** The process according to claim 6, **characterised in that** magnesium chloride is added in a quantity of 10 to 200 mM/l and preferably in a quantity of 50 to 100 mM/l of reaction medium.

8. The process according to claim 3, **characterised in that** the concentration of pure protease in the solution before treatment is from 0.5 to 6 wt.% and particularly about 2.5 wt.%.

9. A process for the preparation of an infant feed based on protein hydrolysate, **characterised in that** a whey product is hydrolysed enzymatically by a purified protease according to claim 1, the hydrolysate is treated at 75-85°C/3-5 min, liquid fat and minerals are added thereto, a UHT treatment is performed at 125-135°C/2-3 min and then carbohydrates, vitamins and trace elements are added, and the liquid product is sterilised by UHT and packaged aseptically.

10. The process according to claim 9, **characterised in that**, after the sterilisation treatment by UHT, the liquid is dried, particularly by spraying.

11. Use of a purified enzymatic preparation according to claim 1 in the preparation of a nutritional, cosmetic or pharmaceutical composition.

**Patentansprüche**

1. Gereinigte proteolytische enzymatische Zubereitung, **dadurch gekennzeichnet, dass** sie eine Phospholipase A2-Restaktivität von höchstens 20 mU/g reinem Enzym besitzt, das durch Analyse mit Hilfe von Hochleistungschromatographie der Phospholipide nach Inkubation mit einer Kleinkinderformulierung nachweisbar ist, deren Phospholipase A2-Aktivität nicht nachweisbar ist, und dass ihre Protease-Aktivität auf mindestens 75 % der Anfangsaktivität des Enzyms gehalten wird.

2. Enzymatische Zubereitung nach Anspruch 1, **dadurch gekennzeichnet, dass** das Enzym Schweinetrypsin ist.

3. Verfahren zur Reinigung von proteolytischem Enzym, insbesondere Trypsin, **dadurch gekennzeichnet,**

   1) **dass** man den pH-Wert einer Lösung der Protease auf einen Wert zwischen 6 und 9 einstellt und dass man die Lösung während mindestens 15 min und höchstens 120 min auf diesem pH-Wert und auf 20-35°C hält, so dass die proteolytische Aktivität der Protease verwendet wird, um die lipolytische Aktivität der Lipasen und der Phospholipasen des Reaktionsmediums zu zerstören, und
   2) **dass** man den pH-Wert der Lösung auf einen Wert kleiner als oder gleich 3,5 senkt, wobei die Reihenfolge der vorhergehenden Schritte 1) und 2) umgekehrt werden kann.

4. Verfahren nach Anspruch 3, **dadurch gekennzeichnet, dass** es einen Endschritt der thermischen Behandlung vorzugsweise durch UHT umfasst, der auch die Beseitigung der Spuren von anderen Restlipasen als der Phospholipase A2 gestattet.

5. Verfahren nach Anspruch 3, **dadurch gekennzeichnet, dass** die Einstellung des pH-Werts zwischen 6 und 9 vor der Senkung des pH-Werts auf einen Wert unter oder gleich 3,5 stattfindet.

6. Verfahren nach Anspruch 3, **dadurch gekennzeichnet, dass** man dem Reaktionsmedium ein in ihm lösliches Magnesiumsalz zusetzt, und zwar vorzugsweise zu Beginn der Reaktion, was die Stabilisierung der Proteasen gestattet und gleichzeitig den Abbau der Phospholipasen begünstigt.

7. Verfahren nach Anspruch 6, **dadurch gekennzeichnet, dass** man Magnesiumchlorid in einer Menge von 10 bis 200 mM/l und vorzugsweise in einer Menge von 50 bis 100 mM/l Reaktionsmedium zusetzt.

8. Verfahren nach Anspruch 3, **dadurch gekennzeichnet, dass** die Konzentration der reinen Protease in der Lösung vor Behandlung zwischen 0,5 und 6 Gew.-% und insbesondere etwa 2,5 Gew.-% beträgt.

9. Verfahren zur Herstellung eines Nahrungsmittels für Kleinkinder auf der Grundlage von Proteinhydrolysat, **dadurch gekennzeichnet, dass** man ein Molkeprodukt enzymatisch mit Hilfe einer gereinigten Protease nach Anspruch 1 hydrolysiert, dass man das Hydrolysat mit 75-85°C/2-5 min behandelt, dass man flüssiges Fett und Mineralstoffe zusetzt, dass man eine UHT-Behandlung bei 125-135°C/2-3 min vornimmt und dass man dann Kohlenhydrate, Vitamine und Oligoelemente zusetzt, dass man das flüssige Produkt durch UHT sterilisiert und keimfrei verpackt.

10. Verfahren nach Anspruch 9, **dadurch gekennzeichnet, dass** man die Flüssigkeit nach der Sterilisationsbehandlung durch UHT insbesondere durch Sprühtrocknung trocknet.

11. Verwendung einer gereinigten enzymatischen Zubereitung nach Anspruch 1 bei der Herstellung einer Nahrungszusammensetzung oder einer kosmetischen oder pharmazeutischen Zusammensetzung.